# EUROPEAN PATENT APPLICATION

(11) **EP 4 831 271 A1**
(43) Date of publication of application: **28.10.2026**
(21) Application number: 23962295.4
(22) Date of filing: 22.12.2023
(51) Int. Cl.: C12M 1/38, C12N 15/10, C12Q 1/6848, C12Q 1/686

(54) **TEMPERATURE CONTROL SYSTEM FOR SAMPLE SOLUTION USED FOR DIGITAL PCR, AND METHOD USING SYSTEM**

(71) Applicant: Hitachi High-Tech Corporation, Minato-ku Tokyo 105-6409 (JP)
(72) Inventor: KAMURA Yoshio, Tokyo 100-8280 (JP); NAKAGAWA Tatsuo, Tokyo 100-8280 (JP)
(74) Representative: MERH-IP Matias Erny Reichl Hoffmann Patentanwälte PartG mbB
(86) International application number: PCT/JP2023/046239
(87) International publication number: WO 2025/134379

(57) **Abstract**

A device includes a first port, a second port, a plurality of chambers, and a flow path that connects the above. The flow path includes: a first flow path that connects the first port and the plurality of chambers; and a second flow path that connects the second port and the plurality of chambers. A flow path resistance of the second flow path is larger than a flow path resistance of the first flow path. A temperature control system seals the plurality of chambers with a separation liquid, and then causes a sample solution in the plurality of chambers to react by using a first temperature controller while pressurizing the first port.

## Description

### Technical Field

The present invention relates to a temperature control system for a sample solution used for digital PCR and a method using the temperature control system for the sample solution in the digital PCR.

### Background Art

In the related art, PCR or real-time PCR has been used for genetic testing. In these techniques, there is a problem that measurement accuracy is low when an amount of a measurement target (nucleic acid) is small. In order to solve this problem, a digital PCR technique has recently attracted attention. In the digital PCR, a sample containing a nucleic acid to be detected is separated into a large number of minute regions, and PCR is performed on each minute region. The type of nucleic acid present in each minute region is determined by determining, based on a fluorescence intensity, a section including the nucleic acid to be detected and a section not including the nucleic acid.

The digital PCR enables highly accurate measurement. On the other hand, since PCR is performed based on one nucleic acid, a fluorescence intensity obtained by the digital PCR is not stronger than a fluorescence intensity obtained by performing PCR in a state where a large amount of nucleic acid is present in a PCR tube as in the real-time PCR in the related art.

PTL 1 discloses, as a DNA detection method using the digital PCR, a DNA detection method for measuring a melting temperature of DNA and a fluorescence labeling probe that hybridizes to the DNA in a droplet containing the DNA and the fluorescence labeling probe.

There are several configurations for sample temperature control in the digital PCR.

PTL 2 discloses a configuration at the time of PCR temperature control in a cartridge-type device holding a through-hole well, and an influence of air bubbles and the like are minimized by tilting a thermal cycler for temperature control.

PTL 3 discloses an apparatus configuration of a microdevice on which a valve is mounted, in which the valve is opened and closed in a process from sample introduction to PCR, the valve is controlled, the sealability of a solution is secured, and an influence of air bubbles is reduced.

PTL 4 discloses a configuration of a microdevice in which a circulation mechanism is mounted, PCR with oil is performed using a circulation apparatus, and air bubbles generated in the microdevice can be washed away in the circulation apparatus.

### Citation List

### Patent Literature

PTL 1: JP2018-108063A
PTL 2: JP2016-518861A
PTL 3: CN107138193A
PTL 4: JP2020-25537A

### Summary of Invention

### Technical Problem

In digital PCR, there is a temperature control process for performing PCR. In addition, in the above-described digital PCR for measuring the melting temperature of DNA, in addition to the temperature control process of PCR, a process of measuring a change in fluorescence intensity while controlling the temperature is added. Therefore, the temperature control process in performing the digital PCR is important.

Since the temperature control is performed from a room temperature to a temperature in the vicinity of 100°C, a sample solution confined in wells in the microdevice is partially evaporated to generate air bubbles. Due to the generation of the air bubbles in the wells, the solution in the wells may push away the oil covering the wells and leak out of the wells. This leads to contamination of surrounding wells.

Since the digital PCR is a highly accurate measurement capable of absolute quantification, the occurrence of such contamination is very problematic. Therefore, it is important to take measures against air bubbles that cause contamination.

Therefore, the tilt of the temperature control mechanism, the mounting of the valve, the introduction of the circulation mechanism, and the like as described above exist as techniques in the related art. However, in these mechanisms, it is necessary to attach a mechanism for preventing air bubbles not only to the device but also to the apparatus side, and not only the cost but also the apparatus size are increased. In addition, in opening and closing of the valve on the chip or opening and closing of the valve of the circulation apparatus, the number of steps in which the apparatus operates also increases. Therefore, it is desirable that a measure against air bubbles during temperature control can be implemented with a simple configuration.

An object of the invention is to provide a method for preventing an influence of air bubbles by generating a flow path resistance in a device of a system, in a sample solution temperature control system in digital PCR.

Another object of the invention is to provide, in the present system, a simple and inexpensive configuration that is the same as an apparatus configuration for introducing a sample solution or dividing a sample without mounting a valve or a circulation apparatus.

The above and other objects and novel features of the invention will become apparent from the description of this specification and the accompanying drawings.

### Solution to Problem

An example of a temperature control system according to the invention is a temperature control system for a sample solution used for digital PCR. The temperature control system includes:
a device, in which
the device includes
   a first port to be pressurized,
   a second port open to an atmosphere
   a plurality of chambers storing a sample solution, and
   a flow path that connects the first port, the second port and the plurality of chambers,
a separation liquid that divides the sample solution flows through the flow path,
the flow path includes
   a first flow path that connects the first port and the plurality of chambers, and
   a second flow path that connects the second port and the plurality of chambers, the temperature control system further includes:
      a pressurizing pump configured to pressurize the device; and
      a first temperature controller configured to control a temperature of the device,
a flow path resistance of the second flow path is larger than a flow path resistance of the first flow path, and
after the plurality of chambers are sealed with the separation liquid, the temperature control system causes the sample solution in the plurality of chambers to react using the first temperature controller while pressurizing the first port.

An example of a method according to the invention is a method using a temperature control system for a sample solution in digital PCR, in which
the temperature control system includes a device,
the device includes
   a first port to be pressurized,
   a second port open to an atmosphere
   a plurality of chambers storing a sample solution, and
   a flow path that connects the first port, the second port and the plurality of chambers,
a separation liquid that divides the sample solution flows through the flow path,
the flow path includes
   a first flow path that connects the first port and the plurality of chambers, and
   a second flow path that connects the second port and the plurality of chambers,
the temperature control system further includes
   a pressurizing pump configured to pressurize the device, and
   a first temperature controller configured to control a temperature of the device,
a flow path resistance of the second flow path is larger than a flow path resistance of the first flow path, and
the method includes:
   a step of sealing the plurality of chambers with the separation liquid; and
   a step of causing the sample solution in the plurality of chambers to react using the first temperature controller while pressurizing the first port after the above step.

### Advantageous Effects of Invention

According to the invention, it is possible to prevent air bubbles in digital PCR with a simple and inexpensive configuration, thereby preventing contamination with adjacent wells.

### Brief Description of Drawings

[FIG. 1] FIG. 1 is a schematic diagram of a pressure gradient generated by disposing a flow path resistance in a device in a temperature control system for a sample solution.
[FIG. 2] FIG. 2 is a plurality of examples of a configuration for generating the flow path resistance in the device in the system.
[FIG. 3] FIG. 3 is a diagram showing a shape of a digital PCR device including a flow path and a well.
[FIG. 4] FIG. 4 is a diagram showing that digital PCR is confirmed by the digital PCR device after introduction of the sample solution, the digital PCR device after introduction of a separation liquid, and temperature control of a thermal cycle, and a configuration diagram thereof.
[FIG. 5] FIG. 5 is a diagram showing a result of digital PCR measurement using the present system.
[FIG. 6] FIG. 6 is a diagram showing a connection (contamination) generated between flow paths when the flow path resistance is insufficient.
[FIG. 7] FIG. 7 is a diagram showing an example in which an influence of the flow path resistance is further strengthened in a micro flow path device.
[FIG. 8] FIG. 8 is a schematic diagram showing that a flow path resistance is generated only by highly viscous oil in the temperature control system for the sample solution.
[FIG. 9] FIG. 9 is a sample temperature control system in which two temperature controllers are combined.
[FIG. 10] FIG. 10 is a reference diagram showing that, in the sample temperature control system, a reverse flow of a sample is generated due to thermal expansion and contraction of a chip or the sample solution, and a connection between flow paths is generated.
[FIG. 11] FIG. 11 is a diagram showing a temperature dependence in oil having a low viscosity or a high viscosity at a room temperature.
[FIG. 12] FIG. 12 is a diagram showing the presence or absence of mixing of oil having a low viscosity or a high viscosity at the room temperature in an atmospheric pressure releasing port.
[FIG. 13] FIG. 13 is a schematic diagram of the device and a diagram showing a shape of a pressurizing port when oil is introduced into the port.
[FIG. 14] FIG. 14 is a diagram showing an example of a configuration in which the sample solution is stored in the device without leaking to the outside at the atmospheric pressure releasing port.
[FIG. 15] FIG. 15 is a diagram showing that melting temperature analysis can also be performed in the sample temperature control system.

### Description of Embodiments

Hereinafter, embodiments of the invention will be described with reference to the drawings.

### [Embodiment 1]

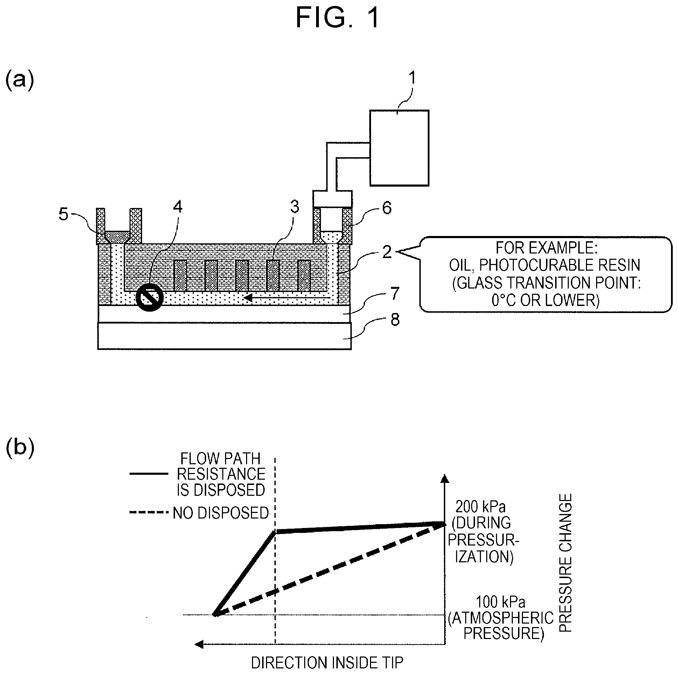

Hereinafter, Embodiment 1 will be described with reference to FIGS. 1 to 5.

Embodiment 1 shows an outline of a pressure gradient generated by disposing a flow path resistance in a device in a temperature control system for a sample solution, and shows that digital PCR can be performed while preventing an influence of air bubbles by applying the pressure gradient to a microdevice. The temperature control system for the sample solution according to the present embodiment is used for the digital PCR, and by using the temperature control system, the digital PCR can be performed with an inexpensive and simple apparatus configuration.

(a) of FIG. 1 shows that a solution in a well can be temperature-controlled while being pressurized by disposing the flow path resistance in the device of the sample temperature control system. The temperature control system includes this device.

The device includes a pressurizing port 6 (a port to be pressurized or a first port) and an atmospheric pressure releasing port 5 (a port open to an atmospheric or a second port). In addition, the temperature control system includes a pressurizing pump 1 for pressurizing the device. In the temperature control during PCR, a separation liquid 2 is continuously introduced from the pressurizing port 6 by the pressurizing pump 1.

At this time, a flow path resistance 4 is disposed in the device. In FIG. 1, the flow path resistance 4 is indicated by a symbol. The atmospheric pressure releasing port 5 is provided on the opposite side of the pressurizing port 6. In addition, a temperature controller 8 (first temperature controller) for controlling the temperature of the device is disposed below a device bottom surface 7.

(b) of FIG. 1 shows a graph of a pressure change in a direction from the port 6 to the port 5 in a chip in the sample temperature control system. When the flow path resistance 4 is not present, the pressure by the pressurizing pump 1 decreases to an atmospheric pressure toward the atmospheric pressure releasing port 5 as indicated by a broken line. On the other hand, when the flow path resistance 4 is present, as indicated by a solid line, the pressure by the pressurizing pump 1 is maintained at substantially the same value until a position where the flow path resistance is present, and when the position of the flow path resistance is exceeded, the pressure decreases to the atmospheric pressure. Therefore, a sample solution 3 confined in the well in the device is continuously pressurized by the pressurizing pump 1.

A boiling point of the sample solution 3 confined in the well is increased by receiving the pressure, and a saturated vapor pressure is increased. This is known in the Clausius-Clapeyron equation. Therefore, in the present system, in the temperature control in the vicinity of 100°C during PCR, generation of air bubbles and the like due to evaporation is prevented in the sample solution 3 confined in the well. As a result, the implementation of the present system leads to the prevention of the sample connection (contamination) in a plurality of adjacent wells.

In the present system, not only the flow path resistance is mounted on a micro flow path device in which 5000 or more wells are present, but also air bubbles are prevented by the system, in which the temperature is controlled while pressurizing, with respect to a process of controlling the temperature in each well unique to the digital PCR. This does not require mounting of a valve, and the atmospheric pressure releasing port 5 may be released to the atmospheric pressure with respect to the pressurizing port 6, which can be implemented by a simple configuration.

At this time, a syringe pump, a diaphragm pump, a compressor, and the like can be used as the pressurizing pump 1. A material of the device may be glass, a resin (cycloolefin polymer, polypropylene, polymethyl methacrylate, polycarbonate, and the like), a semiconductor (silicon and the like), a metal (copper, zinc, and the like), and the like. A cartridge heater, a Peltier element, and the like can be used as the temperature controller 8.

As shown in (a) of FIG. 1, examples of the separation liquid 2 include a photocurable resin and oil. In particular, a liquid having no compatibility with water is preferable because mixing with the sample solution 3 can be avoided. Oil is an example of a suitable separation liquid having low compatibility with water. Examples of the oil include silicone oil, mineral oil, and fluorinated oi.

The flow path resistance includes several elements. The flow path resistance will be described using Equation 1. $Δp = 32 μLu / \left({d}^{2}\right)$ (Equation 1)

This equation is an equation of the flow path resistance (pressure loss) of a pipe flow of a laminar flow. Δp is a flow path resistance, µ is a viscosity, L is a length, u is an average flow velocity, and d is a diameter of a pipe. According to Equation 1, in order to increase the flow path resistance, parameters of the viscosity, the length, and the average flow velocity can be increased, or the diameter of the pipe can be reduced.

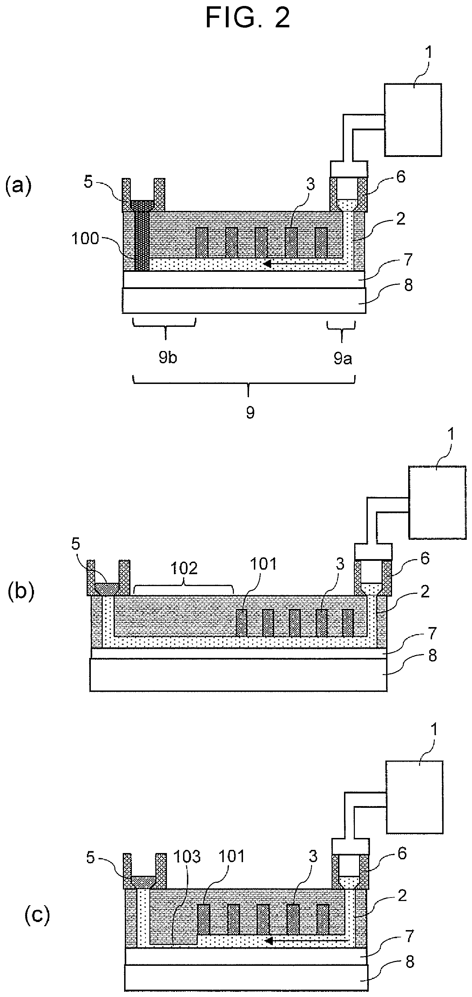

(a) to (c) of FIG. 2 show examples in which the above-described elements are applied to the flow path resistance 4 shown in FIG. 1. The device includes a plurality of wells (a plurality of chambers) that store the sample solution. In addition, the device includes a flow path 9, and the flow path 9 connects the pressurizing port 6, the atmospheric pressure releasing port 5, and the plurality of wells. In particular, the flow path 9 includes a first flow path 9a connecting the pressurizing port 6 and the plurality of wells, and a second flow path 9b connecting the atmospheric pressure releasing port 5 and the plurality of wells.

A flow path resistance of the second flow path 9b is larger than the flow path resistance of the first flow path 9a. Therefore, in a state where the pressurizing port 6 is pressurized, the pressure applied to the first flow path 9a is larger than the pressure applied to the second flow path 9b. Accordingly, the generation of air bubbles in the first flow path 9a is prevented.

In (a) of FIG. 2, a highly viscous separation liquid 100 is disposed in the atmospheric pressure releasing port 5. Therefore, the flow path resistance is increased by a viscosity parameter µ.

In (b) of FIG. 2, a length 102 between the atmospheric pressure releasing port 5 and a well 101 closest to the atmospheric pressure releasing port 5 is increased. Therefore, the flow path resistance is increased by a length parameter L.

In (c) of FIG. 2, a flow path diameter 103 of the flow path between the atmospheric pressure releasing port 5 and the well 101 closest to the atmospheric pressure releasing port 5, that is, the second flow path 9b is narrowed. Therefore, the flow path resistance is increased by a diameter parameter d of the pipe.

The Reynolds number is a guide for the highly viscous separation liquid 100. This will be described using Equation 2. $Re = ρv 1 / μ$ (Equation 2) Re is the Reynolds number, ρ is the density, v is the representative flow velocity, l is the length, and µ is the viscosity. At this time, l corresponds to the diameter of the flow path in the device or the diameter of the atmospheric pressure releasing port 5.

The Reynolds number is a measure representing an influence of the viscosity in the flow. Therefore, when the Reynolds number is large for the separation liquid, it means easy to move, and when the Reynolds number is small, it means difficult to move. That is, considering the flow path resistance described above, the flow path resistance increases as the Reynolds number decreases.

In general, when the Reynolds number is less than 2300, a laminar flow is formed, and when the Reynolds number is larger than 4000, a turbulent flow is formed. In addition, the case of a value therebetween is referred to as a transition. As a guide of the highly viscous separation liquid 100 in the present system, the laminar flow is desirable, and the viscosity preferably has a Reynolds number smaller than 2300.

It is also effective to use a photocurable resin for the separation liquid 2. When the photocurable resin is introduced into the flow path and then photocured, the viscosity can be changed. Therefore, the photocurable resin can be introduced in a low viscosity state at the time of introduction, and the viscosity can be increased by photocuring after the introduction.

The degree of viscosity correlates with a glass transition point (Tg) of the photocurable resin. When a photocurable resin having a Tg of higher than 0°C is photocured, it becomes hard like a resin plate in a normal dPCR environment (about room temperature), and when a photocurable resin having a Tg of 0°C or lower is photocured as shown in (a) of FIG. 1, it becomes like a gel in the normal dPCR environment. As described above, when a photocurable resin having a Tg of 0°C or lower is used, a more suitable viscosity can be implemented.

Examples using these photocurable resins will be described. In this method, when the photocurable resin is introduced regardless of Tg, only the photocurable resin at the same position as that of the highly viscous separation liquid 100 shown in (a) of FIG. 2 is photocured. Accordingly, it is possible to generate a flow path resistance in the vicinity of the atmospheric pressure releasing port 5 and in the flow path to the well.

As described above, the flow path resistance can be increased by some elements. The flow path resistance described above is merely an example, and in addition, the flow path resistance can be increased by bending loss, branch loss, outlet loss, unevenness of the flow path surface, and the like, and a device using these can also be implemented.

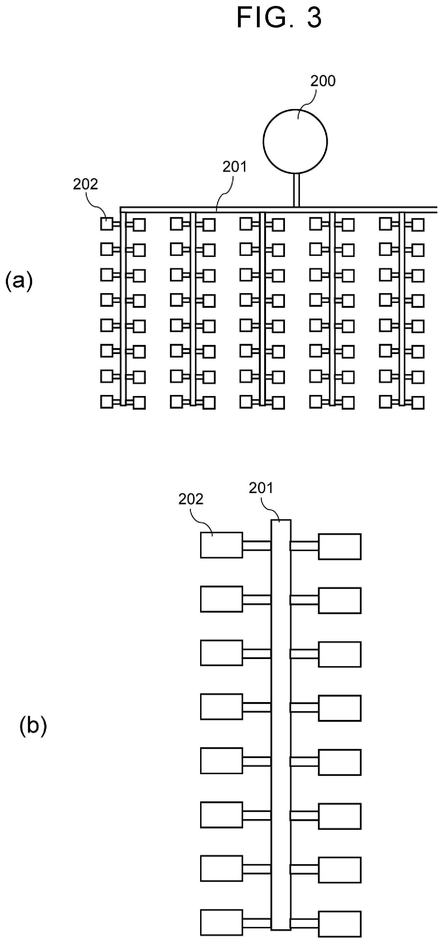

FIG. 3 is a diagram showing a shape of a digital PCR device including a flow path and wells. (a) and (b) of FIG. 3 are diagrams showing a part of the device at a low magnification and a high magnification, respectively.

An inlet portion 200 (first port), a flow path 201, and a plurality of wells 202 (a plurality of chambers) are present in a micro flow path actually used. In FIG. 3, an outlet portion is not particularly shown.

The sample solution is introduced from the inlet portion 200, passes through the flow path 201, and is introduced into the wells 202. The size of the well 202 is wide from a nanometer scale to a micrometer scale, and the shape may be various shapes such as a quadrangle, a circle, and a hexagon. Further, the number of wells 202 is at least one or more, and is at most 3,000,000 or less.

In this device, the flow path resistance can be generated by a branch configuration to each well, a width of the micro flow path itself, a configuration of a curved portion, and the like. Accordingly, the sample solution in each well is pressurized, and air bubbles can be prevented.

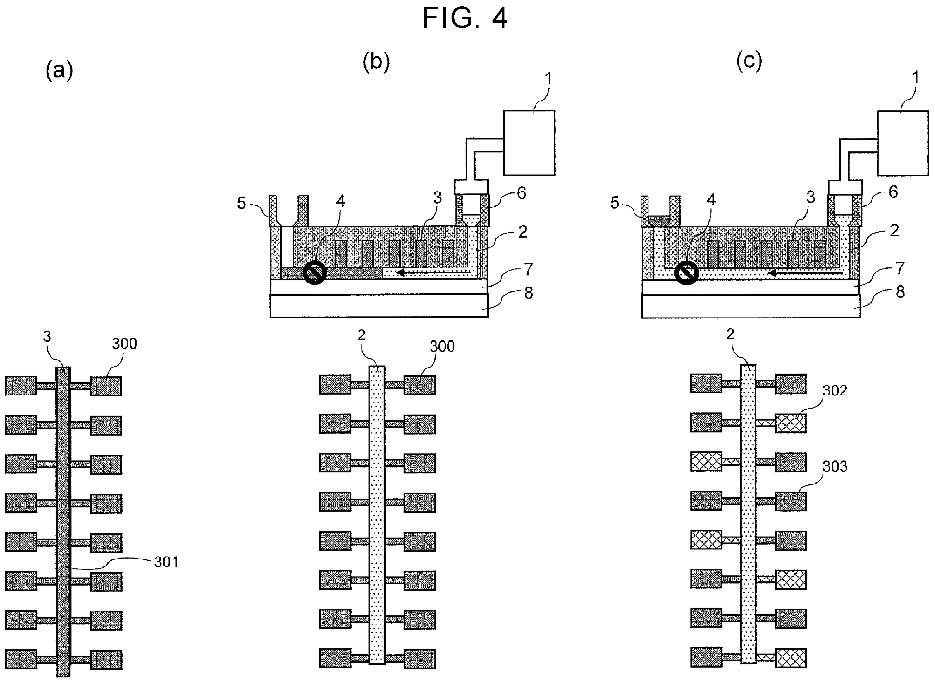

In FIG. 4, (a) of FIG. 4 is a diagram showing the digital PCR device after introduction of the sample solution, (b) of FIG. 4 is a diagram showing the digital PCR device after introduction of the separation liquid, and (c) of FIG. 4 is a diagram showing that digital PCR is confirmed by temperature control of a thermal cycle.

As shown in (a) of FIG. 4, a plurality of wells 300 (a plurality of chambers) and a flow path 301 of the digital PCR device at the time of sample introduction are filled with the sample solution 3. Thereafter, as shown in (b) of FIG. 4, the separation liquid 2 is introduced from the pressurizing port 6 using the pressurizing pump 1. At this time, the sample solution present in the flow path 301 in the device is replaced with the separation liquid 2. Accordingly, the separation liquid 2 that divides the sample solution flows through the flow path 301.

Then, the sample solution is divided into individual wells 300 by the separation liquid 2. The temperature control system photocures the photocurable resin after sealing the wells 300 with the separation liquid and before causing the sample solution to react. Accordingly, sealing and pressurization can be appropriately performed. A person skilled in the art can appropriately design a configuration (light irradiation unit, control unit thereof, and the like) for photocuring the photocurable resin.

(c) of FIG. 4 shows a state where the temperature control of the PCR is performed while pressurizing using the pressurizing pump 1 from the pressurizing port 6 in the same apparatus configuration as that of (b) of FIG. 4. After sealing the wells 300 with the separation liquid 2, the temperature control system causes the sample solution in the wells 300 to react using the temperature controller 8 while pressurizing the pressurizing port 6.

As described above, the present embodiment relates to a method using the temperature control system of the sample solution for the digital PCR. This method includes a step of sealing the wells 300 with the separation liquid 2, and a step of causing the sample solution to react in the wells 300 using the temperature controller 8 while pressurizing the pressurizing port 6.

The pressurizing pump 1 can pressurize the device when the separation liquid 2 is introduced into the device and when the sample solution reacts. Accordingly, the pressurizing pump 1 used to introduce the separation liquid 2 can also be used to prevent air bubbles, which is efficient.

At this time, since the flow path resistance 4 is present in the device, the air bubbles in the sample solution in the wells are continuously prevented. Therefore, the sample solution does not flow out toward the separation liquid 2 present in the flow path 301.

In the evaluation for the digital PCR, fluorescence observation is performed after the temperature control of the PCR. At the time of PCR, the PCR is promoted in a well in which nucleic acid is present, and the PCR does not occur in a well in which nucleic acid is not present. By introducing a fluorescence labeling probe into the sample solution in advance, only wells in which the PCR has occurred emit light during fluorescence measurement.

As shown in (c) of FIG. 4, in the device during fluorescence observation, luminous (positive) wells 302 and non-luminous (negative) wells 303 are observed. From this result, it is determined that the digital PCR was performed without any problem.

As described above, in the present system, there is no change between the system configuration after sample division and the system configuration during PCR, and it is not necessary to add a new apparatus for PCR. Therefore, the configuration is inexpensive, and is simple from the viewpoint of the system configuration and the process of operating the system.

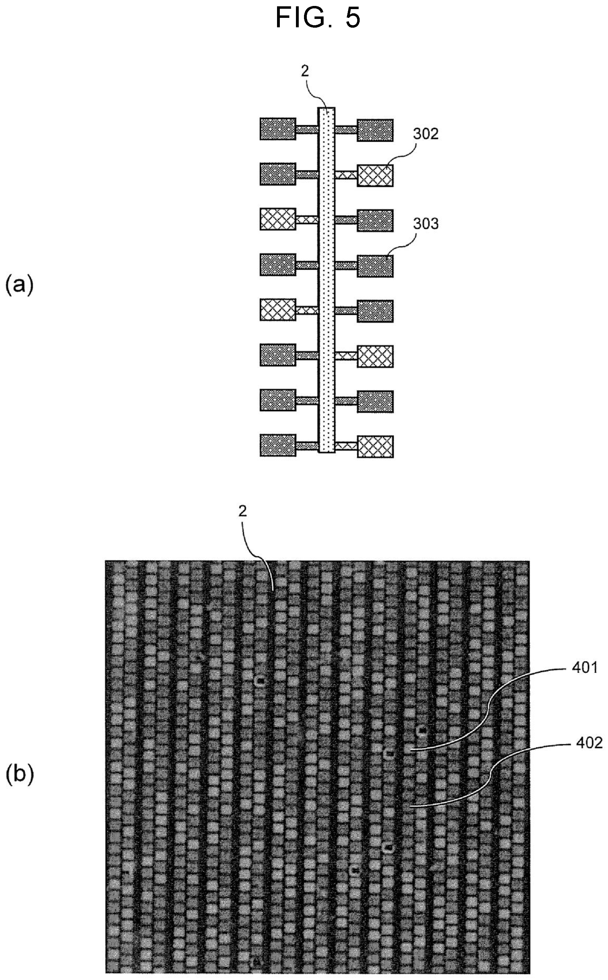

FIG. 5 shows a result of the digital PCR observed by the present system. (a) of FIG. 5 is a schematic cross-sectional view similar to FIG. 4. It can be seen from the fluorescence image shown in (b) of FIG. 5 that luminous (positive) wells 401 and non-luminous (negative) wells 402 are observed in the actual measurement. Therefore, it is found that the digital PCR is performed without any problem, and it is proven that the present system is effective.

### [Embodiment 2]

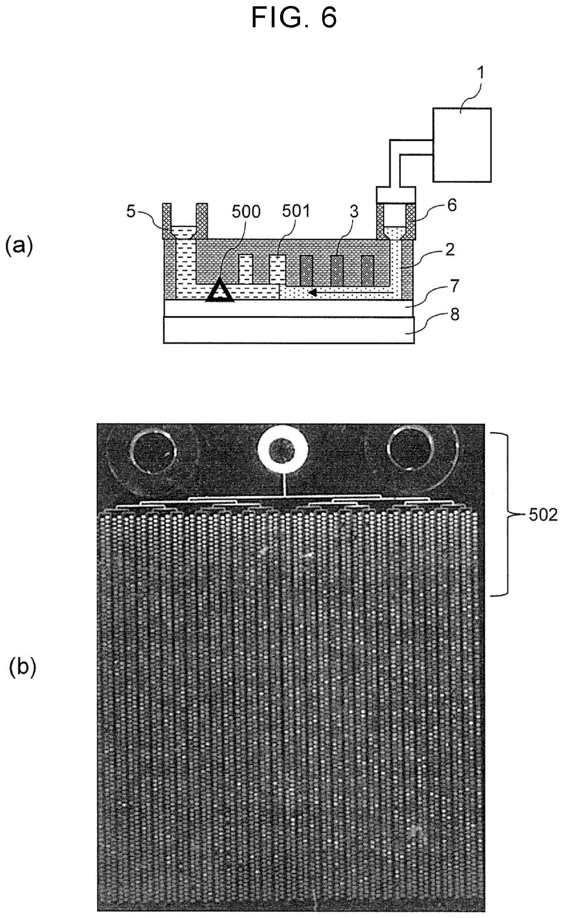

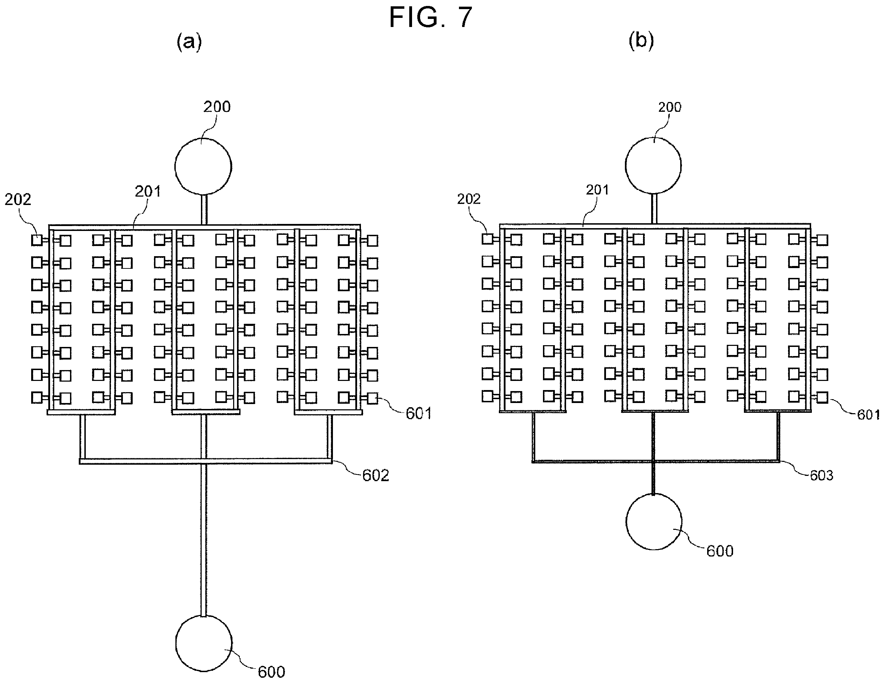

Hereinafter, Embodiment 2 will be described with reference to FIGS. 6 and 7. Embodiment 2 shows a measurement result of the digital PCR observed when the flow path resistance is insufficient, and measures against the flow path resistance.

FIG. 6 shows, as a reference diagram, a result of the digital PCR observed when the flow path resistance is insufficient. (a) of FIG. 6 shows a schematic cross-sectional view similar to FIG. 4, and (b) of FIG. 6 shows an example of a fluorescence image.

When the flow path resistance is an insufficient flow path resistance 500 during temperature control of the PCR, a well 501 in which contamination has occurred is generated in wells close to the atmospheric pressure releasing port 5. This is because the pressurization is not appropriately applied to the well due to the insufficient flow path resistance 500, air bubbles are not prevented, and the sample solution flows out into the flow path. Actually, in an image of the digital PCR, a region 502 in which the contamination has occurred is confirmed.

When the wells are connected to each other as described above, an advantage of the highly accurate digital PCR capable of absolute quantification is impaired. That is, the insufficient flow path resistance 500 lacks the high accuracy of the digital PCR.

Therefore, (a) and (b) of FIG. 7 show an example of a device according to the present embodiment for not leading to an insufficient flow path resistance. In (a) of FIG. 7, a flow path 602 (second flow path) from an outlet portion 600 (second port) on the opposite side of the inlet portion 200 (first port) to a well 601 closest to the outlet portion 600 is lengthened. In (b) of FIG. 7, a flow path diameter of a flow path 603 (second flow path) from the outlet portion 600 on the opposite side of the inlet portion 200 to the well 601 closest to the outlet portion 600 is narrowed.

From the viewpoint of molding a micro flow path chip, it is easy to form the flow path 602 in a tournament shape or a meandering shape. Therefore, it is preferable that the flow path 602 is formed in a tournament shape or a meandering shape and a total length is longer than 0.1 mm from the viewpoint of the flow path resistance. It can be said that the flow path 602 is easily used from the viewpoint of the device size when the total length is shorter than 30 mm.

On the other hand, the flow path diameter of the flow path 603 is limited to several 100 nm to several 10 µm from the viewpoint of processing accuracy. This is a numerical value from the viewpoint of the width. Similarly, from the viewpoint of the depth, several 100 nm to several 10 µm is the limit.

In the present system, the flow path diameter and depth of the microdevice are preferably smaller than 100 µm. When the diameters and depths of the flow path 201 and the flow path 603 are compared, the diameter and depth of the flow path 603 are preferably equal to or less than the diameter and depth of the flow path 201. Similarly, when the total lengths of the flow path 201 and the flow path 602 are compared, it is preferable that the total length of the flow path 602 is equal to or larger than that of the flow path 201.

### [Embodiment 3]

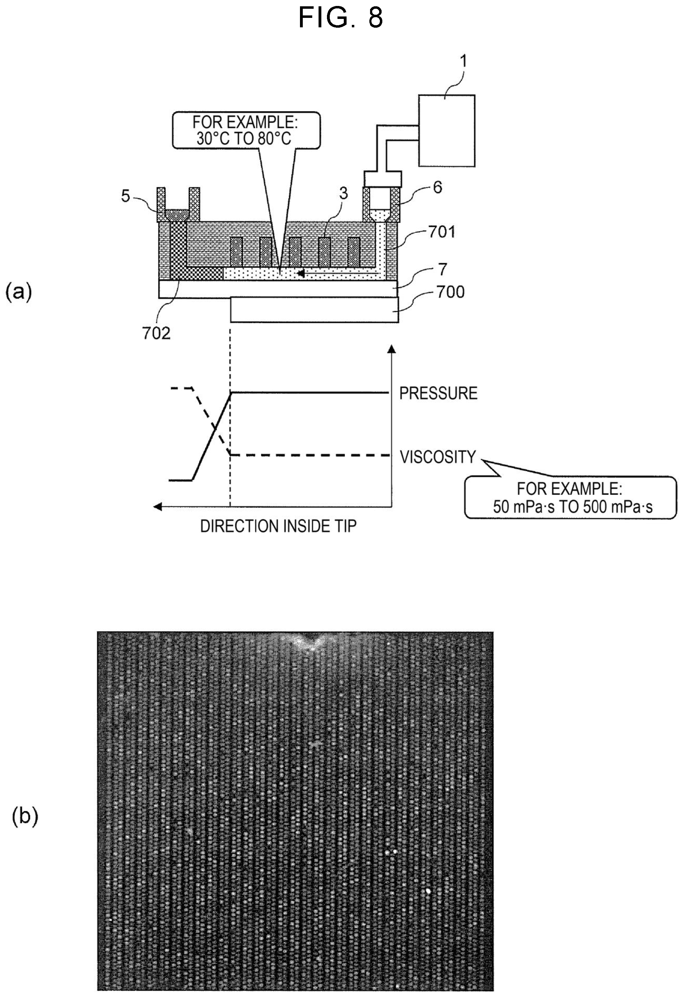

Hereinafter, Embodiment 3 will be described with reference to FIG. 8. FIG. 8 is a diagram showing that it is possible to locally generate a flow path resistance only by using highly viscous oil without devising the shape of the flow path or the like.

A major difference from the system shown in FIG. 1 is that the separation liquid flowing through the flow path is a separation liquid 701 whose viscosity has a temperature dependence, and that the temperature controller is a temperature controller 700 (first temperature controller) that does not heat the vicinity of the atmospheric pressure releasing port 5 and controls the temperature of only the pressurizing port 6 and the well.

An outline of this system will be described with reference to (a) of FIG. 8. By using the pressurizing pump 1, the separation liquid 701 whose viscosity has a temperature dependence is introduced from the pressurizing port 6. At this time, in order to facilitate the introduction of the 701 whose viscosity has a temperature dependence, heating may be performed to about 30°C to 80°C by the temperature controller 700 that controls the temperature of only the pressurizing port 6 and the well. That is, in one example, when the separation liquid 701 is introduced into the device, the temperature controller 700 heats the separation liquid 701 to 30°C to 80°C.

After the separation liquid is moved through the flow path to the atmospheric pressure releasing port 5, the temperature control of PCR is started by the temperature controller 700 that controls the temperature of only the pressurizing port 6 and the well. At this time, the pressurizing pump 1 continues pressurizing as in Embodiment 1.

Since the viscosity of the separation liquid 701, which is present in a region from the pressurizing port 6 to the well and whose viscosity has a temperature dependence, becomes low by such temperature control, the pressure by the pressurizing pump 1 is easily applied. Therefore, the generation of air bubbles in the sample solution 3 in the well is prevented during the temperature control of PCR.

On the other hand, the temperature of the second flow path 9b (see FIG. 2) is lower than the temperature of the temperature controller 700. Therefore, in the vicinity of the atmospheric pressure releasing port 5, which is not heated by the temperature controller, the temperature of the separation liquid 701 whose viscosity has a temperature dependence is not controlled, and thus the temperature of the separation liquid 701 is close to a room temperature and becomes a highly viscous separation liquid 702. Therefore, in this region, the pressure of the pressurizing pump 1 receives the flow path resistance at once and changes to the atmospheric pressure.

(b) of FIG. 8 shows a result of confirming that the digital PCR can be normally executed by actually using this system.

An advantage of the present embodiment is that, unlike Embodiment 1, it is not necessary to generate the flow path resistance by narrowing the flow path or lengthening the flow path. In the digital PCR, increasing the length of the flow path leads to discarding more valuable sample solution (increase in dead volume). In addition, decreasing the length of the flow path has a disadvantage in that it takes time to introduce the sample or the separation liquid. On the other hand, in the present embodiment, the advantage can be achieved only by the separation liquid whose viscosity has a temperature dependence and the devising of the temperature controller.

The separation liquid whose viscosity has a temperature dependence may be a liquid that is not miscible (compatible) with water, such as oil or a photocurable resin. The viscosity is generally from 50 mPa·s to 500 mPa·s at the room temperature. As shown in (a) of FIG. 8, by setting the viscosity of the separation liquid within a range of 50 mPa·s to 500 mPa·s, it is possible to secure an appropriate fluidity. A standard of an upper limit value is calculated from the time for introducing the separation liquid.

Here, a relationship among a flow rate, a pressure, and a viscosity will be described using Equations 3 and 4. $\left(Flow rate\right) ∝ {\left(differential pressure\right)}^{1 / 2}$ (Equation 3) $\left(Flow rate\right) ∝ 1 / viscosity$ (Equation 4)

It is assumed that the separation liquid can be introduced into the device under a certain condition. If the separation liquid needs to be changed to a separation liquid having 4 times the viscosity and the same flow rate needs to be applied to the device, the pressure applied to the device and the separation liquid is 16 times the normal pressure. That is, the pressure required for introduction is proportional to the square of the viscosity. It can also be understood that when the viscosity increases, the flow rate decreases in inverse proportion to the viscosity. That is, the higher the viscosity, the longer the time required for introduction, or the more difficult the introduction.

When there is no upper limit to the time, the separation liquid may be introduced while heating to about 2000 mPa·s. On the other hand, a lower limit value is a condition for sufficiently applying a pressure gradient.

In the example of FIG. 8, no temperature controller is disposed in the atmospheric pressure releasing port 5 and the second flow path 9b. Therefore, the configuration of the temperature control system is further simplified.

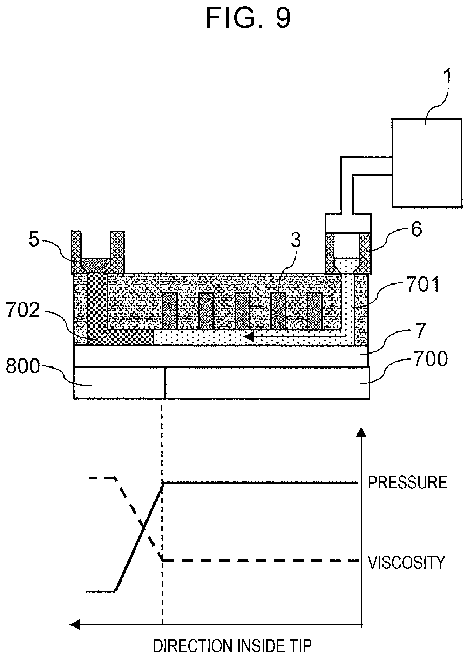

FIG. 9 shows a sample temperature control system in which two temperature controllers are combined as a derivative of the temperature controller of FIG. 8. In FIG. 8, in order to convert the separation liquid 701 whose viscosity has a temperature dependence into the separation liquid 702 whose viscosity is partially high, a part of the separation liquid 701 is not heated, but in FIG. 9, a temperature controller 800 (second temperature controller) for controlling the temperature of the device is newly disposed.

The temperature controller 700 is disposed in the first flow path 9a (see (a) of FIG. 2) (and the pressurizing port 6 and the well in the example of FIG. 9). The temperature controller 800 is disposed in the second flow path 9b (and the atmospheric pressure releasing port 5 in the example of FIG. 9). Since it is possible to cool to a temperature lower than the room temperature by the temperature controller 800, it is possible to achieve a lower temperature and a higher viscosity, and it is possible to improve the degree of the flow path resistance. That is, the temperature controller 700 functions as a heater, and the temperature controller 800 functions as a cooler.

It is also effective to use a photocurable resin for the separation liquid 701. When the photocurable resin is introduced into the flow path and then photocured, the viscosity can be changed. Therefore, the photocurable resin can be introduced in a low viscosity state during introduction, and the viscosity can be increased by photocuring after the introduction.

The degree of the viscosity correlates with the glass transition point (Tg) of the photocurable resin, and a photocurable resin having a Tg exceeding 0°C becomes hard like a resin plate, and a photocurable resin having a Tg of 0°C or lower becomes gel.

Examples using these photocurable resins will be described. When the photocurable resin is introduced regardless of Tg, only the photocurable resin at the same position as the highly viscous separation liquid 702 can be photocured. Accordingly, a flow path resistance can be generated in the second flow path 9b.

Absolutely, Embodiment 3 is merely an example, and various modifications are possible. For example, in consideration of Embodiment 1 and the like, the accuracy of the flow path resistance may be improved by combining the shape of the flow path, the separation liquid whose viscosity has a temperature dependence, the temperature controller that controls the temperature of only the pressurizing port and the well, and the like.

### [Embodiment 4]

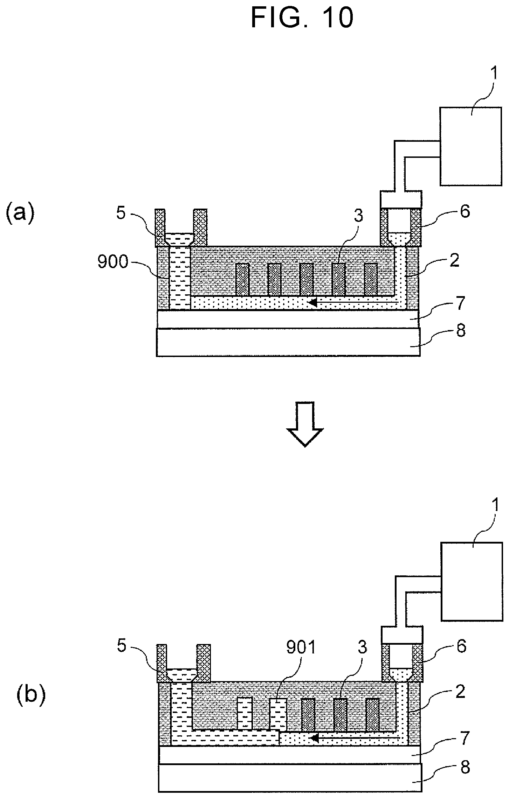

Embodiment 4 will be described below with reference to FIGS. 10, 11, and 12. These drawings show that the sample solution may flow in reverse from the atmospheric pressure releasing port depending on the viscosity of the used separation liquid.

FIG. 10 is a reference schematic view showing a process in which the sample solution flows in reverse. In temperature control during PCR, expansion and contraction of the device, expansion and contraction of air bubbles present at the atmospheric pressure releasing port, and the like occur. These expansion and contraction are main factors, and a mixed solution 900 (see (a) of FIG. 10) of the sample solution and the separation liquid present at the atmospheric pressure releasing port 5 is generated and drawn into the device. As a result, as shown in (b) of FIG. 10, a well 901 in which the sample solutions are connected to each other is generated.

As a solution for preventing the sample reverse flow, the mixed solution 900 of the sample solution and the separation liquid is prevented from being generated. In order not to produce a mixed solution, the Reynolds number shown in the above Equation 2 may be used. The Reynolds number is a measure representing an influence of the viscosity in the flow. Therefore, when the Reynolds number is large for the separation liquid, it means easy to move, and when the Reynolds number is small, it means difficult to move. That is, as the Reynolds number is smaller, the separation liquid tends to form a laminar flow and is difficult to mix.

In general, when the Reynolds number is less than 2300, a laminar flow is formed, and when the Reynolds number is larger than 4000, a turbulent flow is formed. In addition, the case of a value therebetween is referred to as a transition. In the present embodiment, it is desirable that the separation liquid 2 is a laminar flow, and therefore, the viscosity at which the Reynolds number is less than 2300 is good.

It is also effective to use a photocurable resin for the separation liquid 2. When the photocurable resin is introduced into the flow path and then photocured, the viscosity can be changed. Therefore, the photocurable resin can be introduced in a low viscosity state at the time of introduction, and the viscosity can be increased by photocuring after the introduction.

The degree of the viscosity correlates with the glass transition point (Tg) of the photocurable resin, and a photocurable resin having a Tg exceeding 0°C becomes hard like a resin plate, and a photocurable resin having a Tg of 0°C or lower becomes gel. Therefore, it is possible to obtain the same effect by using a photocurable resin that becomes highly viscous by photocuring. For example, a photocurable resin having a Tg of 0°C or lower has a viscosity close to that of gel. Therefore, the Reynolds number becomes lower than 2300, and a laminar flow is formed.

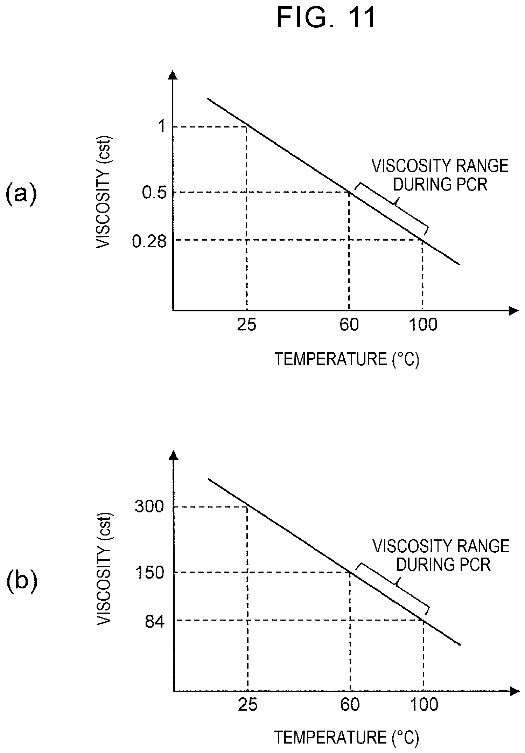

FIG. 11 shows a graph in which the viscosity of the separation liquid changes according to the temperature. (a) and (b) of FIG. 11 show a separation liquid having a low viscosity and a separation liquid having a high viscosity at the room temperature, respectively.

As shown in FIG. 10, the viscosity of the separation liquid present in the atmospheric pressure releasing port 5 decreases with heating during temperature control of PCR. Therefore, when the viscosity becomes low, the Reynolds number becomes large, and mixing with the sample solution becomes easy. Therefore, in order to prevent mixing with the sample solution not only at the room temperature but also at the temperature control of PCR, it is preferable that the viscosity of the separation liquid at the room temperature is high as shown in (b) of FIG. 11. In the example of (b) of FIG. 11, the viscosity of the separation liquid at the room temperature (for example, 25°C) is 300 cst, and the viscosity of the separation liquid during execution of PCR is 60 cst to 100 cst.

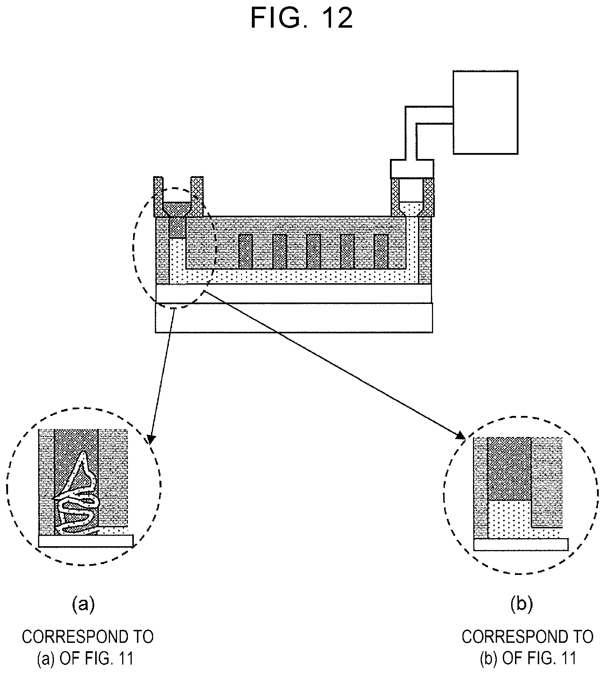

(a) and (b) of FIG. 12 schematically show the states of the sample solution and the separation liquid present in the atmospheric pressure releasing port 5 when the separation liquid having a low viscosity and the separation liquid having a high viscosity at the room temperature in (a) and (b) of FIG. 11 are used. When the separation liquid of (b) of FIG. 11 is used, the separation liquid becomes a laminar flow at the atmospheric pressure releasing port 5 as shown in (b) of FIG. 12.

Summarizing the contents described above, in order to prevent the sample solution and the separation liquid from being mixed with each other, it is desirable that the condition is satisfied such that the separation liquid becomes a laminar flow, and it is preferable that the condition at the time of heating during PCR is also satisfied. Accordingly, it is possible to prevent a reverse flow of the sample solution. Further, from the viewpoint of the viscosity of the separation liquid that becomes a laminar flow, it is expected to increase the effect of preventing the reverse flow by devising conditions of a temperature control cycle during the PCR. For example, a temperature cycle at the time of temperature increase and/or temperature decrease during the PCR may be delayed as a whole (< 10°C/sec), or a temperature cycle at the time of temperature decrease may be delayed from that at the time of temperature increase. These contribute to reducing (slowing down) the degree (speed) of expansion and contraction of air bubbles and the like. Accordingly, it is possible to reduce an amount of the mixed solution 900 of the sample solution and the separation liquid drawn into the device.

### [Embodiment 5]

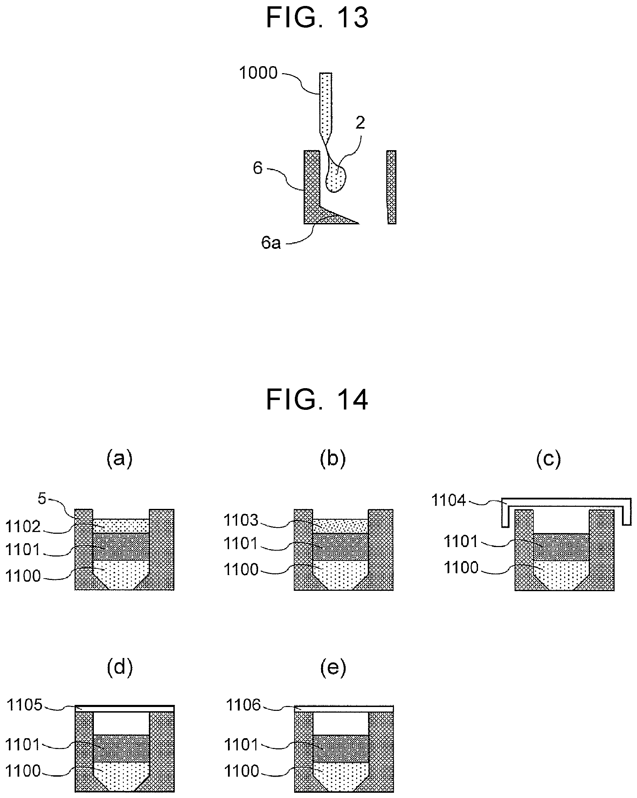

Hereinafter, Embodiment 5 will be described with reference to FIGS. 13 and 14. These drawings are diagrams showing the configurations of the atmospheric pressure releasing port and the pressurizing port.

FIG. 13 shows an example of the shape of the pressurizing port 6. The separation liquid 2 is introduced into the port before pressurization is performed from the pressurizing port 6. At this time, it is desirable to prevent air and the like from entering the separation liquid when the separation liquid is introduced. Therefore, the pressurizing port 6 may be provided with a tapered portion 6a to have a shape that does not allow air to easily enter.

In addition, the pressurizing port 6 may be surface-treated in advance such that a contact angle between the separation liquid 2 and a wall surface of the pressurizing port 6 is reduced. It is also possible to take measures by a method of injecting the separation liquid 2, instead of taking measures against mixing of air by the structure of the pressurizing port 6 side. For example, a dispenser 1000 for the separation liquid may be devised such that the separation liquid 2 is introduced along the wall surface of the pressurizing port 6 during introduction.

FIG. 14 is a diagram showing a configuration for preventing the sample solution from being eluted to the outside of the device through the atmospheric pressure releasing port 5.

In the present embodiment, the system is provided such that pressurization is continued during temperature control in PCR. Therefore, the separation liquid and the sample solution present deeper than the separation liquid are continuously pushed. At this time, when the sample solution adheres (contaminates) to the outside of the device, for example, an experimental environment or the inside of the apparatus at a certain moment, this leads to carryover. That is, when the same measurement is performed using another device after such contamination, measurement data becomes inaccurate. Therefore, it is desirable that the sample solution is not exposed as much as possible to the atmospheric pressure releasing port.

Therefore, configurations as shown in (a) to (e) of FIG. 14 are conceivable. These drawings show a state where a separation liquid 1100 and a sample solution 1101 are accumulated in the atmospheric pressure releasing port 5 after PCR. While the atmospheric pressure is released, measures are taken on an upper portion of the sample solution to prevent the sample solution from flowing out of the device.

In (a) of FIG. 14, oil 1102 is disposed on the sample solution 1101. The oil may be the same as the separation liquid 1100. The oil is preferably lighter than the sample solution, and in particular, the density of the oil is preferably smaller than that of the sample solution. In order to avoid mixing with the sample solution, the oil is preferably hydrophobic.

In (b) of FIG. 14, a photocurable resin 1103 is disposed on the sample solution 1101. Similarly, the photocurable resin 1103 preferably has a density lower than the sample solution 1101. The photocurable resin is hydrophobic and is preferably not mixed with the sample solution. The photocurable resin 1103 may be cured after PCR and used as a solid lid.

In (c) of FIG. 14, a lid 1104 with a gap is disposed. Since there is a pressure releasing cap as a commercially available product, a lid having such a configuration may be disposed.

In (d) of FIG. 14, an air-permeable film 1105 is disposed. Examples of the air-permeable film include PTFE and PDMS, and it is desirable that the air-permeable film has a water repellency.

In (e) of FIG. 14, an absorbent material 1106 is disposed. Examples of the absorbent material include absorbent cotton, and the excess sample solution 1101 is absorbed while securing air permeability.

Ideally, the lid, the air-permeable film, the absorbent material, the oil, and the photocurable resin are desirably disposed before the start of PCR. Various methods are conceivable for a disposing timing.

The first case is an example in which the sample solution is introduced from the atmospheric pressure releasing port 5, and the oil or photocurable resin may be disposed on the sample solution in advance. In this state, evacuation may be performed from the pressurizing port 6 to introduce the sample from the atmospheric pressure releasing port 5 toward the pressurizing port 6, or a pressurizing pump may be connected to the atmospheric pressure releasing port 5 to introduce the sample from the atmospheric pressure releasing port 5 toward the pressurizing port 6. The configuration shown in (a) and (b) of FIG. 14 is established by introducing the separation liquid from the pressurizing port 6 after the sample solution is introduced.

It is also possible to introduce the sample solution from the pressurizing port 6. At that time, the oil or photocurable resin, the sample solution, and the separation liquid are disposed in this order from below in the pressurizing port 6, the sample is introduced from the pressurizing port 6 by the pressurizing pump, and then the sample is divided, whereby the configuration shown in (a) and (b) of FIG. 14 is established.

In the examples of (a) and (b) of FIG. 14, in the temperature control system, after the wells are sealed with the separation liquid 1100 and before the sample solution 1101 reacts, the photocurable resin 1103 or the oil 1102 can be disposed in the atmospheric pressure releasing port 5. Accordingly, the pressure control can be performed at an appropriate timing.

The temperature control system can be implemented to automatically dispose the photocurable resin 1103 or the oil 1102. In addition, specific units (fluid providing unit, control unit thereof, and the like) for disposing the photocurable resin 1103 or the oil 1102 can be appropriately designed by a person skilled in the art.

In the examples of (c) to (e) of FIG. 14, in the temperature control system, after the wells are sealed with the separation liquid 1100 and before the sample solution 1101 reacts, the lid 1104 (pressure releasing cap), the water-repellent air-permeable film 1105, or the absorbent material 1106 can be disposed in the atmospheric pressure releasing port 5. Accordingly, the pressure control can be performed at an appropriate timing.

The temperature control system can be implemented to automatically dispose the lid 1104, the air-permeable film 1105, or the absorbent material 1106. Specific units (gripping unit, conveying unit, control unit thereof, and the like) for disposing these members in the atmospheric pressure releasing port 5 can be appropriately designed by a person skilled in the art.

### [Embodiment 6]

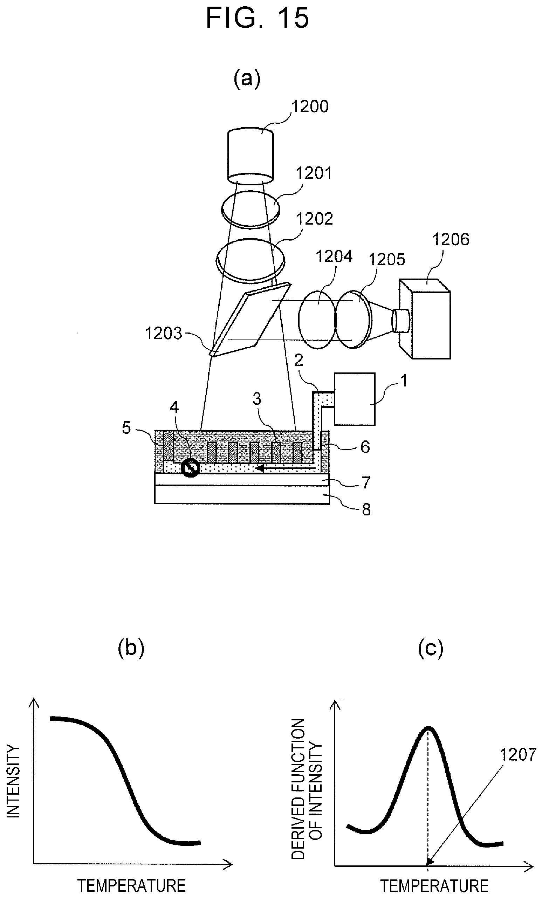

Hereinafter, Embodiment 6 will be described with reference to FIG. 15. FIG. 15 is a diagram showing that a melting temperature of the sample solution can be analyzed using the present system.

As a DNA detection method using the digital PCR, there is a DNA detection method for measuring a melting temperature of DNA and a fluorescence labeling probe that hybridizes to the DNA in a droplet containing the DNA and the fluorescence labeling probe. (a) of FIG. 15 shows an apparatus configuration for performing the melting temperature analysis.

An optical system for fluorescence measurement is prepared above the temperature control system. In the fluorescence measurement, a light having a necessary wavelength is extracted from an excitation light 1200 by an excitation light filter 1201, and the extracted light is focused on a sample by a lens 1202. The light extracted from the excitation light 1200 transmits through a dichroic half mirror 1203. The fluorescence emitted from the sample is reflected. The fluorescence from the sample passes through a fluorescence filter 1204 and is then imaged on a photodetector 1206 by an imaging lens 1205.

In this fluorescence measurement, the melting temperature of the DNA can be calculated by acquiring a fluorescence image while the temperature controller 8 is heating or cooling at a constant ramp rate.

(b) of FIG. 15 shows a graph of changes in temperature and fluorescence intensity in the melting temperature analysis. When the fluorescence intensity change is acquired while heating, a monotonous sigmoid curve is obtained. An inflection point of the sigmoid curve corresponds to the melting temperature of the DNA. Therefore, the fluorescence intensity is differentiated with respect to the temperature based on the sigmoid curve ((c) of FIG. 15), and a melting temperature 1207 can be calculated as a peak value.

Accordingly, the present sample temperature control system can be used in combination with the optical system. Unlike the temperature control during the PCR, the temperature control in the melting temperature analysis does not heat to near 100°C. Therefore, a pump mechanism such as pressurization may be removed during the fluorescence measurement.

As described above, the temperature control system can perform the fluorescence measurement (for example, melting temperature measurement) accompanied by temperature control using the temperature controller 8. Accordingly, the fluorescence measurement can be appropriately executed.

The invention is not limited to the embodiments described above, and includes various modifications. For example, the embodiments described above have been described in detail to facilitate understanding of the invention, and the invention is not necessarily limited to those including all the configurations described above. A part of a configuration of a certain embodiment can be replaced with a configuration of another embodiment, and a configuration of another embodiment can be added to a configuration of a certain embodiment. A part of a configuration in each embodiment may be added to, deleted from, or replaced with another configuration.

### Reference Signs List

- 1: pressurizing pump
- 2: separation liquid
- 3: sample solution
- 4: flow path resistance
- 5: atmospheric pressure releasing port (second port)
- 6: pressurizing port (first port)
- 6a: tapered portion
- 7: device bottom surface
- 8: temperature controller (first temperature controller)
- 9: flow path
- 9a: first flow path
- 9b: second flow path
- 100: highly viscous separation liquid
- 101: well closest to atmospheric pressure releasing port
- 102: length between atmospheric pressure releasing port and well closest to port
- 103: flow path diameter between atmospheric pressure releasing port and well closest to port
- 200: inlet portion (first port)
- 201: flow path
- 202: well (a plurality of chambers)
- 300: well (a plurality of chambers)
- 301: flow path
- 302: luminous (positive) well
- 303: non-luminous (negative) well
- 401: luminous (positive) well in actual measurement
- 402: non-luminous (negative) well in actual measurement
- 500: insufficient flow path resistance
- 501: well in which contamination occurred
- 502: region in which contamination occurred
- 600: outlet portion (second port)
- 601: well closest to outlet portion
- 602: flow path (second flow path) from outlet portion to well closest to outlet portion
- 603: flow path (second flow path) from outlet portion to well closest to outlet portion
- 700: temperature controller (first temperature controller)
- 701: separation liquid whose viscosity has temperature dependence
- 702: highly viscous separation liquid
- 800: temperature controller (second temperature controller)
- 900: mixed solution of sample solution and separation liquid
- 901: well in which sample solutions are connected to each other
- 1000: dispenser
- 1100: separation liquid
- 1101: sample solution
- 1102: oil
- 1103: photocurable resin
- 1104: lid (pressure releasing cap)
- 1105: air-permeable film
- 1106: absorbent material
- 1200: excitation light
- 1201: excitation light filter
- 1202: lens
- 1203: dichroic half mirror
- 1204: fluorescence filter
- 1205: imaging lens
- 1206: photodetector
- 1207: melting temperature

## Claims

1. A temperature control system for a sample solution used for digital PCR, the temperature control system comprising:
a device, wherein
the device includes
a first port to be pressurized,
a second port open to an atmosphere ,
a plurality of chambers storing a sample solution, and
a flow path that connects the first port, the second port and the plurality of chambers,
a separation liquid that divides the sample solution flows through the flow path,
the flow path includes
a first flow path that connects the first port and the plurality of chambers, and
a second flow path that connects the second port and the plurality of chambers, the temperature control system further comprises:
a pressurizing pump configured to pressurize the device; and
a first temperature controller configured to control a temperature of the device,
a flow path resistance of the second flow path is larger than a flow path resistance of the first flow path, and
after the plurality of chambers are sealed with the separation liquid, the temperature control system causes the sample solution in the plurality of chambers to react using the first temperature controller while pressurizing the first port.

2. The temperature control system according to claim 1, wherein
a viscosity of the separation liquid has a temperature dependence.

3. The temperature control system according to claim 2, wherein
the first temperature controller heats the separation liquid to 30°C to 80°C when the separation liquid is introduced into the device.

4. The temperature control system according to claim 1, wherein
a pressure applied to the first flow path is higher than a pressure applied to the second flow path.

5. The temperature control system according to claim 1, wherein
a temperature of the second flow path is lower than a temperature of the first temperature controller.

6. The temperature control system according to claim 5, wherein
no temperature controller is disposed in the second port and the second flow path.

7. The temperature control system according to claim 5, further comprising:
a second temperature controller configured to adjust a temperature of the device, wherein
the first temperature controller is disposed in the first flow path, and
the second temperature controller is disposed in the second flow path.

8. The temperature control system according to claim 1, wherein
the pressurizing pump is configured to
when the separation liquid is introduced into the device, and
when the sample solution is caused to react,
pressurize the device.

9. The temperature control system according to claim 1, wherein
the separation liquid becomes a laminar flow at the second port.

10. The temperature control system according to claim 9, wherein
a viscosity of the separation liquid is in a range of 50 mPa·s to 500 mPa·s.

11. The temperature control system according to claim 1, wherein
the separation liquid has no compatibility with water.

12. The temperature control system according to claim 11, wherein
the separation liquid is oil.

13. The temperature control system according to claim 11, wherein
the separation liquid is a photocurable resin.

14. The temperature control system according to claim 13, wherein
the photocurable resin has a glass transition point of 0°C or lower.

15. The temperature control system according to claim 13, wherein
the temperature control system photocures the photocurable resin before causing the sample solution to react.

16. The temperature control system according to claim 1, wherein
the temperature control system disposes a photocurable resin or oil in the second port before causing the sample solution to react.

17. The temperature control system according to claim 16, wherein
the photocurable resin or the oil has a density lower than that of the sample solution and is hydrophobic.

18. The temperature control system according to claim 1, wherein
the temperature control system disposes a pressure releasing cap or a water-repellent air-permeable film or an absorbent material on the second port before causing the sample solution to react.

19. The temperature control system according to claim 1, wherein
fluorescence measurement with temperature control is performed using the temperature controller.

20. A method using a temperature control system for a sample solution in digital PCR, wherein
the temperature control system includes a device,
the device includes
a first port to be pressurized,
a second port open to an atmosphere,
a plurality of chambers storing a sample solution, and
a flow path that connects the first port, the second port and the plurality of chambers,
a separation liquid that divides the sample solution flows through the flow path,
the flow path includes
a first flow path that connects the first port and the plurality of chambers, and
a second flow path that connects the second port and the plurality of chambers,
the temperature control system further includes
a pressurizing pump configured to pressurize the device, and
a first temperature controller configured to control a temperature of the device,
a flow path resistance of the second flow path is larger than a flow path resistance of the first flow path, and
the method comprises:
a step of sealing the plurality of chambers with the separation liquid; and
a step of causing the sample solution in the plurality of chambers to react using the first temperature controller while pressurizing the first port after the above step.
